# EUROPEAN PATENT APPLICATION

(11) **EP 3 028 719 A1**
(43) Date of publication of application: **08.06.2016**
(21) Application number: 14195663.1
(22) Date of filing: 01.12.2014
(51) Int. Cl.: A61K 47/48, A61L 31/10, A61L 31/16

(54) **Polymer-drug conjugate based on a polyisoolefin-based copolymer**

(71) Applicant: Lanxess Inc., Sarnia, Ontario N7T 7M2 (CA); The University of Western Ontario, London, ON N6G 4X8 (CA)
(72) Inventor: Gilles, Elizabeth R., London, Ontario N6G 1Z1 (CA); McEachran, Matthew, Sarnia, Ontario N7T 6A3 (CA); Trant, John Frederick, London, Ontario L6Y 5N9 (CA); Sran, Inderpreet, Brampton, Ontario L5Y 5N9 (CA); Ferrari, Lorenzo, Burlington, Ontario L7L 7MB (CA)
(74) Representative: Deblon, Jörg-Stephan

(57) **Abstract**

A polymer-drug conjugate, which can be used in medical applications such as stents, has at least one active agent conjugated through a carboxylic acid moiety to a copolymer derived from at least one isoolefin monomer and at least one copolymerizable monomer, where the copolymerizable monomer is at least one multiolefin monomer, a β-pinene monomer or a mixture thereof. Such conjugates show improved adhesion to stainless steel and a substantial decrease in burst release of paclitaxel form a drug eluting stent (DES).

## Description

### Field

This application relates to polymer-drug conjugates, particularly to conjugates of a drug with a carboxylic acid functionalized polyisoolefin-based copolymer.

### Background

Butyl rubber is a synthetic elastomer, which is used in many high performance applications due to its many attractive properties. These properties include water/gas impermeability, chemical stability, high elasticity and biocompatibility. Commercial butyl rubber is a co-polymer of isobutylene with small amounts of isoprene (IP). Butyl rubber and its co-polymers are important components in many commercial products such as inner liners for automobile tires, sporting equipment, sealants and even chewing gum.

There has been some recent development in polyisobutylene-based materials for a number of biomedical applications, including polyisobutylene-co-polystyrene (SIBS) triblock copolymers, which are currently being used for drug eluting coatings on vascular stents. This class of polymers has also been investigated as synthetic aortic valves and as shunts for glaucoma. However, even the commercialized polymers have exhibited problems when integrated into implants. For example, SIBS was investigated as an implant in the urinary tract but there was significant attachment of uropathogenic species such as *E. coli* 67. Also, in the stent application there was coating delamination *in vivo.* Another major issue related to drug release applications, in particular for stents, is burst-release of the physically encapsulated drug from the laminate surface causing a decrease in the life of the stent and/or thrombotic events.

There remains a need for new materials better suited for medical applications, including controlled drug release applications.

### Summary

In one aspect, there is provided a polymer-drug conjugate comprising at least one active agent conjugated through a carboxylic acid moiety to a copolymer derived from at least one isoolefin monomer and at least one copolymerizable monomer, the copolymerizable monomer comprising at least one multiolefin monomer, a β-pinene monomer or a mixture thereof.

There is also provided a use of a therapeutically effective amount of the polymer-drug-conjugate for treating or preventing a disease or condition in a subject.

There is also provided a use of a therapeutically effective amount of the polymer-drug-conjugate for manufacturing a medicament for treating or preventing a disease or condition in a subject.

There is also provided a method of treating or preventing a disease in a subject comprising administering a therapeutically effective amount of the polymer-drug-conjugate to a subject in need of the treatment or prevention.

There is also provided a functionalized copolymer comprising at least one free carboxylic acid (-COOH) group bonded to a copolymer of at least one isoolefin monomer and at least one copolymerizable monomer, the copolymerizable monomer comprising at least one multiolefin monomer, a β-pinene monomer or a mixture thereof.

There is also provided a film of the polymer-drug conjugate or the functionalized copolymer deposited on a substrate.

There is also provided a stent comprising a mesh tube coated with the polymer-drug conjugate.

Polymer-drug conjugates of the present disclosure may help control drug release in medical applications. Introduction of carboxylic acid groups on to the polyisoolefin-based copolymer may serve as a site for immobilization (e.g. covalent immobilization) of drugs. Immobilization may occur through chemical linkages, for example ester or amide linkages, in particular ester linkages. Immobilization helps reduce burst release of the drug, creating a slower more controlled release of the drug. Further, free carboxylic acid groups (-COOH) on the copolymer may serve to enhance adhesion to materials, for example metals and bone, which would mitigate against delamination of the copolymer from the material, and would be beneficial in various applications including stents (e.g. drug eluting stents (DES)), bone cements and the like.

The polymer-drug conjugates are particularly useful for vascular stent coatings. Vascular stents are introduced to narrow, diseased peripheral or coronary arteries to prevent stenosis (obstruction of blood flow). Drug-eluting stents slowly release drug molecules to block cell proliferation, which reduces the possibility of fibrosis, which together with thrombus (clots) could otherwise block the stented artery (restenosis).

Further features will be described or will become apparent in the course of the following detailed description. It should be understood that each feature described herein may be utilized in any combination with any one or more of the other described features, and that each feature does not necessarily rely on the presence of another feature except where evident to one of skill in the art.

### Brief Description of the Drawings

For clearer understanding, preferred embodiments will now be described in detail by way of example, with reference to the accompanying drawings, in which:

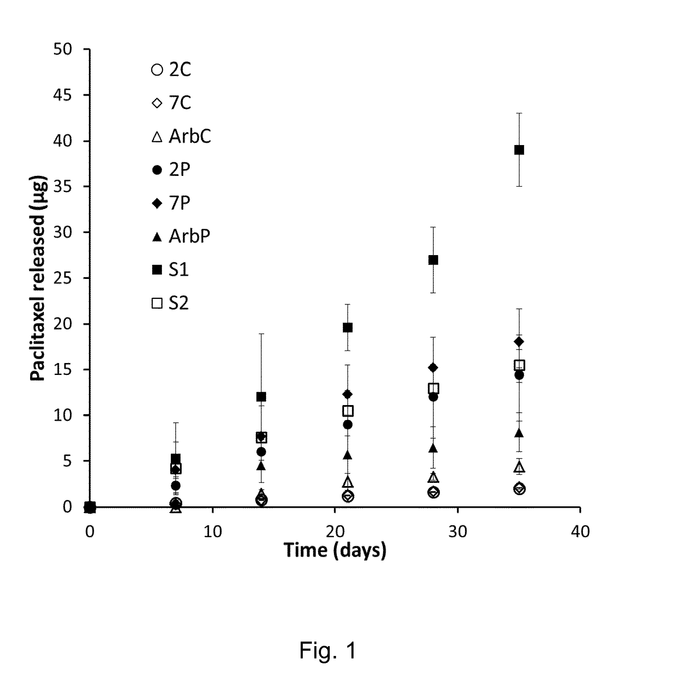

Fig. 1 depicts a graph of mass released (µg) vs. time (days) for the release of paclitaxel (PTx) from polymeric substrates, where 2C, 7C and ArbC are polymer-drug conjugates of the present invention, 2P, 7P and ArbP are comparable polymer-drug compositions in which the paclitaxel is physically encapsulated in the carboxylic acid functionalized butyl rubber instead of being conjugated to the butyl rubber, and S1 and S2 are SIBS-paclitaxel compositions in which the paclitaxel is physically encapsulated in the SIBS. Note that 2C and 7C overlap for much of the time course.

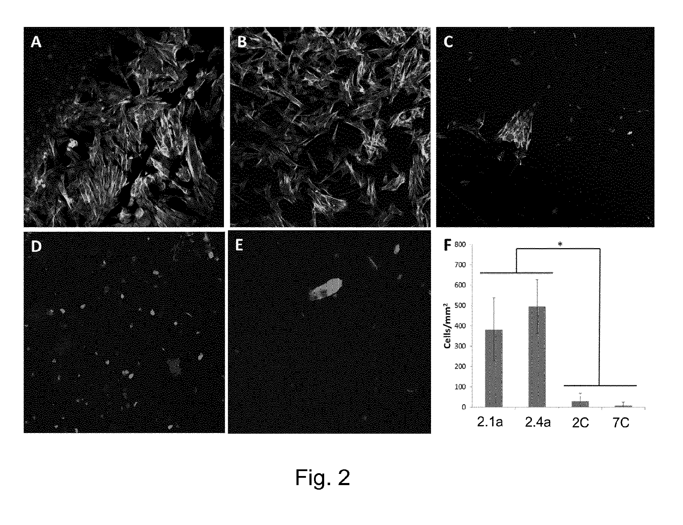

Fig. 2 shows confocal microscopy images of C2C12 cells on: A) 2.1 a; B) 2.4a; C) 2C, showing a rare region with cells adhered; D) 2C, showing a more typical region of the surface; E) 7C; F) Cells/mm² for the different films showing statistically reduced numbers of cells on 2C and 7C. (*P < 0.05) The cell nuclei are stained with DAPI and the cytoskeletons are stained with Alexa Fluor 568 phalloidin. All images are the same magnification and each image represents an area of 0.4 mm x 0.4 mm.

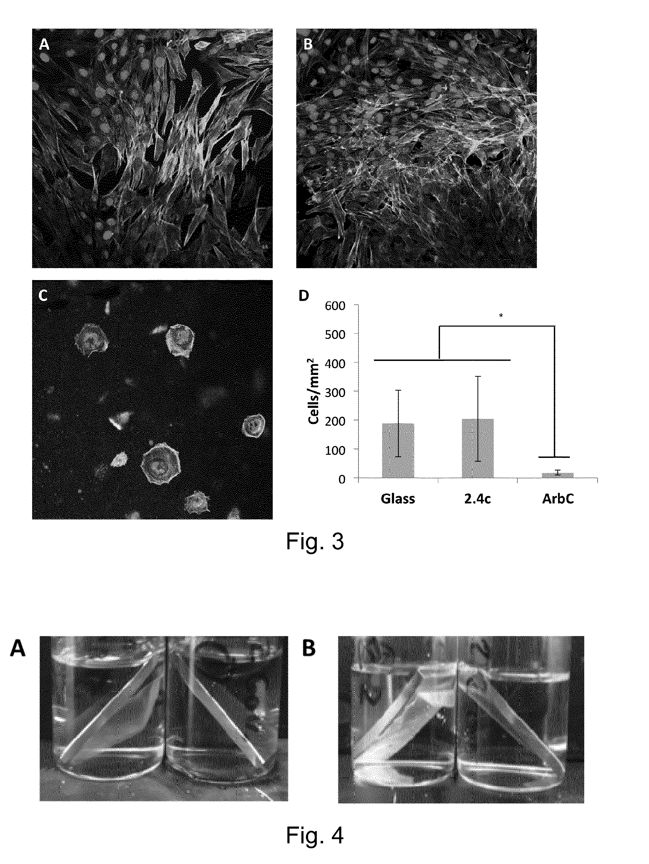

Fig. 3 shows confocal microscopy images of C2C12 cells on: A) glass slide; B) 2.4c; C) ArbC; D) Cells/mm² for the different films showing statistically reduced numbers of cells on ArbC. (*P < 0.05) The cell nuclei are stained with DAPI and the cytoskeletons are stained with Alexa Fluor 568 phalloidin. All images are the same magnification and each image represents an area of 0.4 mm x 0.4 mm.
Fig. 4 shows the reduced film delamination for the drug conjugates: A) left beaker contains S1 after 35-day treatment with complete delamination; right beaker contains 2C at 35 days with minimal delamination B) left beaker contains 7P at 35 days showing slight delamination; right beaker contains 7C at 35 days showing no delamination.

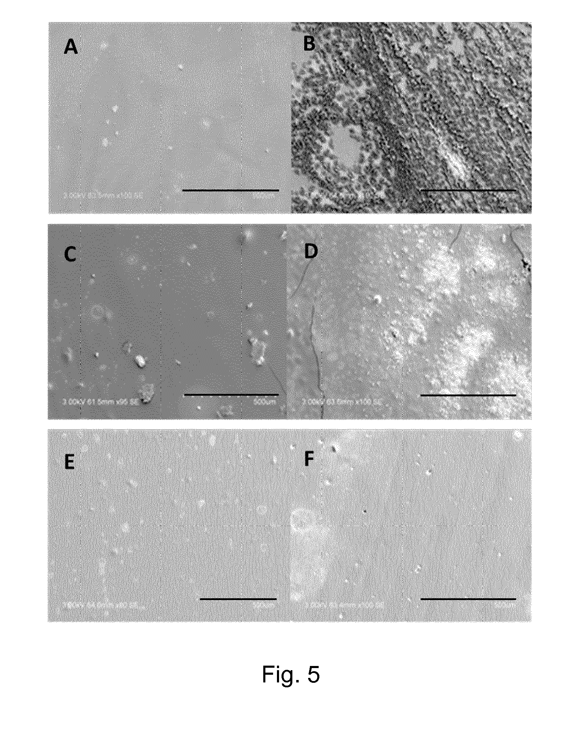

Fig. 5 shows SEM images of polymer-PTX films which reveal gross structural changes due to the release study. A, C and E were taken prior to release; B, D and F were taken after release. A, B) S1; C, D) 2P; E, F) 2C. 100 x magnification (except C, 95 x magnification, and E, 80 x magnification); scale bars represent 500 µm.

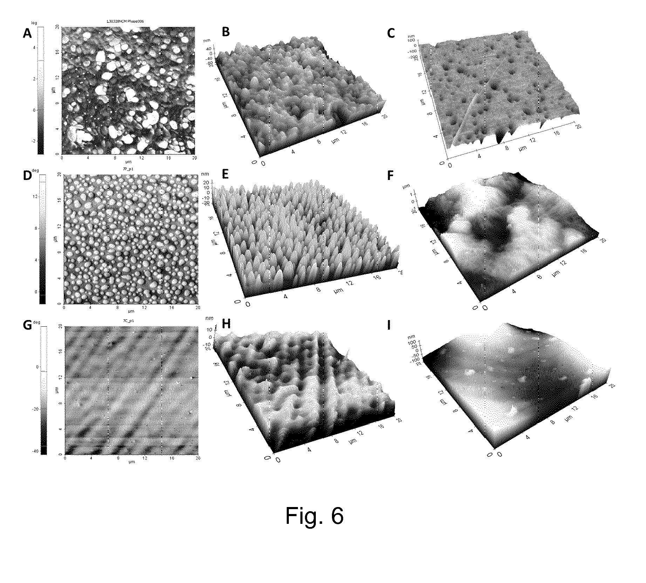

Fig. 6 shows examples of AFM images showing the polymer surface before and after the release study: A-C) S1; D-F) 7P; G-H) 7C. Phase images: A, D, G; topography images before PTX release: B, E, H; topography images after 35 days of PTX release: C, F, I.

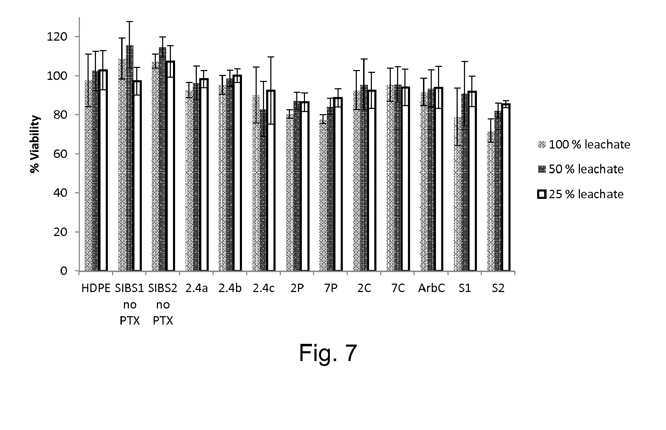

Fig. 7 depicts a graph showing results of an MTT assay for evaluating toxicity of the various polymers with and without paclitaxel, where: HDPE = high density polyethylene as a control; SIBS1 and SIBS2 are styrene-isobutylene-styrene triblock copolymers containing 10 and 20% polystyrene respectively; 2.4a-c are carboxylic acid functionalized polymers prepared from 2.2 mol%, 7 mol%, and 6 mol% arborescent rubbers respectively (defined as in Scheme 2); 2P = carboxylic acid functionalized butyl rubber (2.2 mol% IP) with 24 wt% physically immobilized paclitaxel; 2C = carboxylic acid functionalized butyl rubber (2.2 mol% IP) with about 24 wt% covalently conjugated paclitaxel; 7P = carboxylic acid functionalized butyl rubber (7 mol% IP) with 48 wt% physically immobilized paclitaxel; 7C = carboxylic acid functionalized butyl rubber (7 mol% IP) with about 48 wt% covalently conjugated paclitaxel; ArbC = carboxylic acid functionalized arborescent butyl rubber (6 mol% IP) with about 38 wt% covalently conjugated paclitaxel; S1 = SIBS1 with 24 wt% physically immobilized paclitaxel; S2 = SIBS2 with 48 wt% physically immobilized paclitaxel.

### Detailed Description

The polymer-drug conjugate comprises a butyl rubber polymer. Butyl rubber polymers are copolymers derived from at least one isoolefin monomer and at least one copolymerizable monomer, the copolymerizable monomer comprising at least one multiolefin monomer, a β-pinene monomer or a mixture thereof.

The butyl rubber polymer is not limited to a special polyisoolefin. However, polyisoolefins produced from isoolefin monomers having from 4-16 carbon atoms, preferably 4-7 carbon atoms, such as isobutene, 2-methyl-1-butene, 3-methyl-1-butene, 2-methyl-2-butene, 4-methyl-1-pentene and mixtures thereof are preferred. More preferred is isobutene (IB, also called isobutylene).

The copolymerizable monomer provides unsaturation in the copolymer. The butyl rubber polymer is not limited to a specific multiolefin. Multiolefins copolymerizable with the isoolefins, as known to one skilled in the art, can be used in the practice of the present invention. Conjugated diene multiolefin monomers are preferred. Examples of such multiolefins include, for example, those having in the range of from 4-14 carbon atoms. Examples of suitable multiolefins include isoprene, butadiene, 2-methylbutadiene, 2,4-dimethylbutadiene, piperyline, 3-methyl-1,3-pentadiene, 2,4-hexadiene, 2-neopentylbutadiene, 2-methly-1,5-hexadiene, 2,5-dimethly-2,4-hexadiene, 2-methyl-1,4-pentadiene, 2-methyl-1,6-heptadiene, cyclopentadiene, methylcyclopentadiene, cyclohexadiene, 1-vinyl-cyclohexadiene and mixtures thereof. A preferred multiolefin comprises isoprene (IP).

The level of unsaturation in the copolymer arises from the amount of the monomer that provides unsaturation (e.g. β-pinene or at least one multiolefin) present in the copolymer. The level of unsaturation based on moles of monomer is preferably about 50% or less. The level of unsaturation may be in a range of about 0.1-50 mol%, more preferably about 0.2-30 mol%, yet more preferably about 0.5-12 mol%, yet more preferably about 2-8 mol%. In some embodiments, by controlling the multiolefin and/or β-pinene content of the butyl rubber polymer, the extent of carboxylic acid functionalization can be advantageously controlled. Through control of carboxylic acid functionalization, drug release and/or surface adhesion properties of the polymer-drug conjugate may be tuned.

Optionally, the butyl rubber polymer may include a co-monomer other than the above referenced multiolefins or β-pinene, such as an alkyl-substituted vinyl aromatic co-monomer, including but not limited to a C₁-C₄ alkyl substituted styrene. Specific examples of such co-monomers include, for example, α-methyl styrene, p-methyl styrene, chlorostyrene, cyclopentadiene, methylcyclopentadiene, indene and mixtures thereof. In one embodiment, the butyl rubber polymer may include, for example, random copolymers of isobutylene, isoprene and para-methylstryene.

The butyl rubber architecture may be linear or arborescent. Arborescent polymers comprise graft polymers comprising a dendritic (multilevel)-branched architecture, typically resulting from successive grafting reactions of linear chain segments on substrates having randomly distributed coupling sites.

The polymer-drug conjugate comprises a carboxylic acid moiety. The carboxylic acid moiety may be polymeric, oligomeric or non-polymeric, preferably non-polymeric. The carboxylic acid moiety is more preferably a C₁₋₁₀ organic residue comprising a carboxyl group. The organic residue may comprise one or more heteroatoms, for example O, S, Cl or Br. Preferably, the carboxylic acid moiety is a C₁₋₁₀ organic residue comprising a carboxyl group and further O atoms. The carboxylic acid moiety is more preferably a residue of a ring-opened cyclic anhydride, preferably a saturated cyclic anhydride. The cyclic anhydride before ring-opening may have comprised, for example, a four-, five-, six-, seven- or eight-membered ring, preferably a five- or six-membered ring. Particular examples of residues of cyclic anhydrides include a residue of diglycolic anhydride, pentandioic anhydride (glutaric anhydride) or succinic anhydride. Residues of diglycolic anhydride are particularly preferred. In a particularly preferred embodiment, the polymer-drug conjugate has Formula (I):

Polymer-Rc-Ag (I)

wherein Polymer is the copolymer derived from at least one isoolefin monomer and at least one copolymerizable monomer, Ag is the active agent, and Rc comprises a ring-opened residue of a saturated cyclic anhydride. Rc is preferably 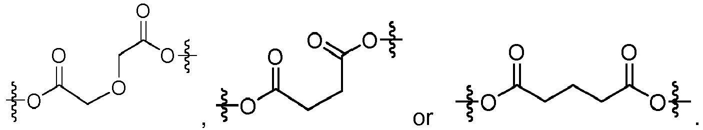 In a particularly preferred embodiment, Rc is 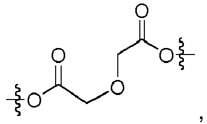 which comprises a residue of diglycolic anhydride.

The polymer-drug conjugate comprises at least one active agent. Any active agent, such as a small molecule drug or a biomolecular drug, may be delivered using the polymer-drug conjugate of the present disclosure. In some embodiments, at least one active agent is a biologically active compound, for example peptides, proteins, therapeutic agents, diagnostic agents, non-biological materials, or combinations thereof. The active agent may be any physiologically or pharmacologically active substance that can produce a desired biological effect, for example an effect that treats or prevents a disease or condition in a subject. The active agent may be, for example, a chemotherapeutic agent, an immunosuppressive agent, a cytokine, a cytotoxic agent, a nucleolytic compound, and a pro-drug enzyme, which may be naturally occurring or produced by synthetic or recombinant methods or combination thereof.

Active agents that are affected by classical multi-drug resistance, such as vinca alkaloids (e.g., vinblastine, vincristine), the anthracyclines (e.g., doxorubicin and daunorubicin), RNA transcription inhibitors (e.g., actinomycin-D), and microtubule stabilizing drugs (e.g., paclitaxel) are of particular note as the active agent. In some embodiments, the active agent may be a hydrophobic drug or a hydrophilic drug. A cancer chemotherapy agent may be a preferred active agent. In an embodiment, the active agent is paclitaxel. In another embodiment, the active agent is rapamycin.

To produce the polymer-drug conjugate, the copolymer may be first functionalized with a carboxylic acid group to form a carboxylic acid functionalized copolymer, and then the carboxylic acid functionalized copolymer contacted with the active agent to form the conjugate. The carboxylic acid functionalized copolymer may possess sufficient carboxylic acid groups to accommodate interaction with the amount of active agent desired in the conjugate, and to have free carboxylic acid groups (-COOH groups) left over to enhance adhesion of the conjugate to a substrate.

Carboxylic acid functionalized copolymers may be formed by any suitable method. In one embodiment, an ethylenically unsaturated carboxylic acid may be grafted on to the copolymer chain in a free radical initiated process, as described in US 2009/189118, for example. In another embodiment, the copolymer may be epoxidized at allylic moieties (multiolefin units) followed by acidification to yield an allylic alcohol functionalized copolymer, and thereafter the allylic alcohol converted to a carboxylic acid in any number of ways including ring opening additions of cyclic anhydrides, thiol-ene click reactions and ring-opening polymerizations (ROP) of cyclic carbonates followed by de-protection to produce multi-acid functionalized copolymers. In one embodiment, carboxylic acid functionalized copolymers may comprise carboxylic acid moieties as described above that are protonated instead of bound to the active agent. In a preferred embodiment, the functionalized copolymers comprise free carboxylic acid groups comprising a ring-opened residue of a saturated cyclic anhydride, for example 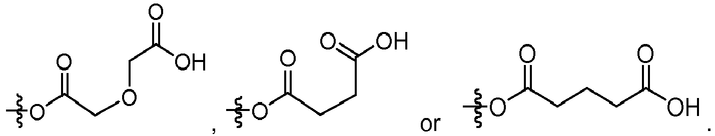 In a particularly preferred embodiment, the free carboxylic acid group is 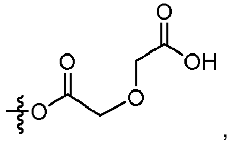 which comprises a residue of diglycolic anhydride.

The active agent should possess a functional group capable of interacting with the carboxylic acid group on the copolymer. The interaction may be through any type of bonding, for example, ionic, covalent or hydrogen bonding. In some embodiments, the one or more drug molecules may be attached to the copolymer via a covalent linkage, in which case the functional group on the active agent that reacts with the carboxylic acid group may be, for example, a hydroxyl or an amine group. Catalysts may be employed to assist reactions between the carboxylic acid group on the copolymer and the functional group on the active agent. Dehydrating agents (e.g. carbodiimides) may also be employed to create the linkages (e.g. ester bonds) through conjugation. Preferably, a hydroxyl group on the active agent reacts with a free carboxylic acid group on the copolymer to form an ester bond. The copolymer may be conjugated with any number of active agent molecules. In particular, it is to be understood that the conjugate may include a single drug molecule or a plurality of drug molecules. In some embodiments, the covalent linkage of the active agent with the copolymer is via a cleavable covalent bond. The cleavable bond may be cleavable in response to an environmental condition within a target cell, for example pH, temperature, etc.

The target cell in a subject may be, for example, a cancer cell, in particular a therapy-resistant cancer cell. In various embodiments, the cancer may be at least one of breast cancer, lung cancer, prostate cancer, ovarian cancer, brain cancer, liver cancer, cervical cancer, bone cancer, esophageal cancer, bladder cancer, uterine cancer, testicular cancer, leukemia, lymphoma, stomach cancer, pancreatic cancer, cancer of smooth muscles (e.g. vascular smooth muscles) or combinations thereof. The subject is preferably mammalian, for example humans, dogs, cats, horses, mice, rats, guinea pigs, monkeys and the like.

The polymer-drug conjugate may be administered to the subject in any suitable manner. Administration routes may include, for example, orally; through injection by SC, IV, intraperitoneal, intracerebral (intraparenchymal), intracerebroventricular, intramuscular, intraocular, intraarterial, intraportal, or intralesional routes; by sustained release systems; or by implantation devices (e.g. stents). Where desired, the compositions can be administered by bolus injection or continuously by infusion, or by implantation device. A therapeutically effective amount of the polymer-drug conjugate may be administered as a single dose, as two or more doses (which may or may not contain the same amount of the desired active agent molecule) over time, or as a continuous infusion via an implantation device or catheter. The dosage level will depend on the active agent and the subject and can be determined by the skilled physician on a case-by-case basis.

In one embodiment, the polymer-drug conjugate may be coated on an implantation device, for example a stent (e.g. a vascular stent), and the device implanted in the subject. Coating of the polymer-drug conjugate may involve creating a film of the polymer-drug conjugate on a substrate. Substrates may comprise any material suitable for the application in which the substrate is being used. Some materials include steel (e.g. stainless steel), ceramic, glass and polymers or polymer composites. In stent applications, the material is preferably stainless steel. Coatings of the polymer-drug conjugate on stainless steel may have an adhesion force as measured by an adhesion test procedure based upon ASTM D-429 Method A of about 15 psi or greater, preferably about 25 psi or greater, or even 30 psi or greater.

### EXAMPLES

### Materials and Methods:

LANXESS Butyl 402 (M_{w} = 4.69 x 10⁵ g/mol, PDI = 2.4, polymer 2.1 a) and a butyl rubber containing 7 mol% isoprene (M_{w} = 1.05 x 10⁶ g/mol, PDI = 3.3, polymer 2.1 b) were obtained from LANXESS Inc. Arborescent poly(isobutylene-co-isoprene) with 6 mol% isoprene (M_{w} = 216 kDa, PDI = 1.4) was prepared as previously reported (Puskas 2009) and provided by LANXESS Inc. Paclitaxel was purchased from LC laboratories (Woburn, Ma). Solvents were purchased from Caledon and all other chemicals were purchased from Sigma-Aldrich and were used without further purification unless otherwise noted Dry toluene was obtained from a solvent purification system. ¹H NMR spectra were obtained in CDCl₃ at 400 MHz. NMR chemical shifts (δ) are reported in ppm and are calibrated against residual solvent signals of CDCl₃ (δ 7.26). Infrared spectra were obtained as films from CH₂Cl₂ on NaCl plates using a Bruker Tensor 27 instrument. Size exclusion chromatography (SEC) was performed in THF using a Waters 515 pump, Wyatt Rex differential refractometer, and two PolyPore columns (300 x 7.5 mm², Agilent) connected in series. Calibration was performed using polystyrene standards. Differential scanning calorimetry (DSC) and thermal gravimetric analysis (TGA) was performed on a Mettler Toledo DSC 822e at a heating rate of 10 °C/min from -120 to +150 °C.

### Synthesis of Allylic Alcohol Functionalized Butyl Rubber.

Allylic alcohol functionalized butyl rubber serves as a starting point for producing carboxylic acid functionalized butyl rubber. To produce the allylic alcohol functionalized butyl rubber, butyl rubber is first epoxidized by prior art methods (Jian 1991; Puskas 1994), and then the epoxide is treated with acid by prior art methods (Bonduelle 2010) to form the allylic alcohol. As shown in Scheme 1, upon treatment of butyl rubber (2.1a-c) with m-chloroperoxybenzoic acid (m-CPBA), epoxide functionalized butyl rubber (2.2a-c) is produced. The epoxide can then be treated with aqueous hydrochloric acid to provide allylic alcohol functionalized butyl rubber (2.3a-c).

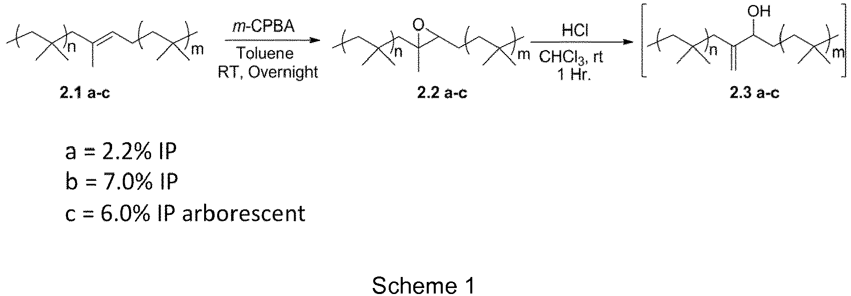

Three types of allylic alcohol functionalized butyl rubber were produced, one with 2.2 mol% isoprene (2.3a), one with 7.0 mol% isoprene (2.3b) and one arborescent with 6.0 mol% isoprene (2.3c).

### Synthesis of Carboxylic Acid Functionalized Butyl Rubber Using Cyclic Anhydrides:

As shown in Scheme 2, the allylic alcohol functionalized butyl rubbers (2.3a-c) may be converted to carboxylic acid functionalized butyl rubbers (2.4-2.6a-c) by ring-opening attack of various cyclic anhydrides in the presence of triethylamine (TEA).

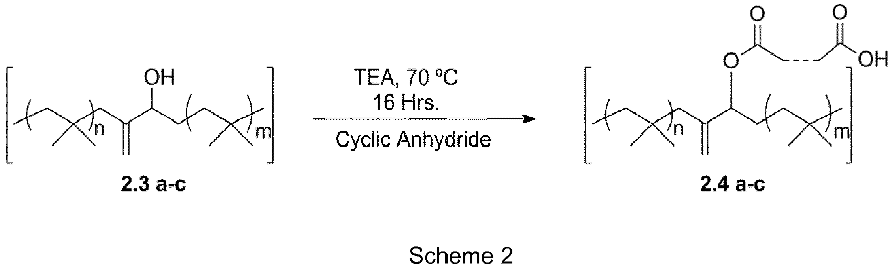

Table 1 provides the structures of the cyclic anhydrides used, the number of equivalents used in the syntheses provided below and the percent conversion to final product. Polymers 2.5 and 2.6 were made similarly to polymer 2.4a.

**Table 1**

| Anhydride | Polymer | Equivalents | % Conversion |
|---|---|---|---|
| diglycolic anhydride | 2.4a-c | 10-20 | >95 |
| 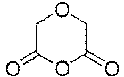 | | | |
| pentandioic anhydride | 2.5 | 20 | -50 |
| 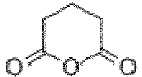 | | | |
| succinic anhydride | 2.6 | 20 | 5-6 |
| 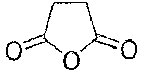 | | | |

### Synthesis of polymer 2.4a

Allylic alcohol functionalized butyl rubber (2.3a) (10 g, 3.9 mmol of -OH) was dissolved in 350 mL of toluene. The solution was heated to 70°C, then 20 equivalents of triethylamine (10.9 mL, 78 mmol) was added followed by 2 equivalents of 4-(dimethylamino)pyridine (0.99 g, 7.8 mmol). A solution of diglycdic anhydride (10 equivalents, 4.5 g, 39 mmol) dissolved in toluene was then added via syringe and the reaction mixture was stirred at 70°C overnight. The product was then washed with distilled water and 6 M HCl twice, followed by concentration under reduced pressure. The product (polymer 2.4a) was further purified by precipitation (2:1 Acetone/Toluene) and then dried under vacuum. Conversion = >95%. Yield = 90%. ¹H NMR (400 MHz, CDCl₃) 5.29 (br s, 1H), 5.12 (s, 1H), 4.95 (s,1H), 4.20-4.40 (m, 4H), 1.42 (s, 145H), 1.12 (s, 431 H). SEC: M_{w} = 308,900 g/mol, PDI = 2.52. IR: 1230, 1365. 1390, 1475, 1733, 1758, 2974 cm⁻¹. T_{g} = -61 °C.

### Synthesis of polymer 2.4b

Epoxide functionalized butyl rubber (2.2b) (10 g, 12.1 mmol of epoxide) was dissolved in 350 mL toluene. The solution was treated with one equivalent HCl (1.0 mL, 12.1 mmol) and allowed to react at room temperature for 1 hour. Due to solubility issues allylic alcohol functionalized butyl rubber 2.3b was not isolated. Instead, the HCl was neutralized with sodium carbonate and then dried with MgSO₄. The mixture was then centrifuged and 2.3b was decanted from the MgSO₄. The solution was heated to 70°C, then 20 equivalents of triethylamine (33.7 mL, 242 mmol) was added followed by 2 equivalents of 4-(dimethylamino)pyridine (3.1 g, 24.2 mmol). A solution of diglycolic anhydride (10 equivalents, 14.0 g, 121 mmol) dissolved in toluene was then added via syringe and the reaction mixture was stirred at 70°C overnight. The product was then washed with distilled water and 6 M HCl twice, followed by concentration under reduced pressure. The product was further purified by precipitation (2:1 Acetone/Toluene) and then dried under vacuum. Conversion = 100%. Yield = 90%. ¹H NMR (400 MHz, CDCl₃) 5.29 (brs, 1H), 5.12 (s, 1H), 4.95 (s,1H), 4.20-4.40 (m, 4H), 1.42 (s, 69H), 1.12 (s, 209H). IR: 1230, 1365. 1390, 1475, 1733, 1758, 2974 cm⁻¹. T_{g} = -53°C.

### Synthesis of polymer 2.4c

Allylic alcohol functionalized polymer (2.3c) (7.0 g, 7.3 mmol of alcohol) was dissolved in toluene (300 mL). The toluene and any residual water were then removed by azeotropic evaporation and replaced with fresh anhydrous toluene (300 mL). Diglycolic anhydride (17 g, 150 mmol) was added along with freshly distilled triethylamine (21 mL, 150 mmol) and 4-(dimethylamino)aminopyridine (DMAP) (1.8 g, 15 mmol). The reaction mixture was heated at 70 °C for 36 h, and then was cooled to ambient temperature. It was then washed with 1 M HCl, saturated NaHCO₃, and water. The reaction was then precipitated into acetone (1 L), redissolved in toluene (250 mL) and reprecipitated into acetone (1 L) to provide polymer (2.4c) (7.0 g, > 98%) as an off-white rubbery solid. T_{g} = -66 °C; ¹H NMR^{*} (600 MHz, CDCl₃) δ 5.29-5.21 (m, 0.02H), 5.13-5.10 (m, 0.02H), 4.97-4.85 (m, 0.04H), 4.32-4.16 (m, 0.53H), 1.41 (s, 29H), 1.10 (s, 88H); IR (thin film on NaCl, chloroform): 923, 950, 1228, 1367, 1390, 1486, 1756, 3006 cm⁻¹. SEC: M_{w} = 228 kDa, PDI = 1.6.

### Adhesion of Carboxylic Acid Functionalized Butyl Rubber to Substrates:

An issue surrounding butyl rubber as a biomaterial is delamination of butyl rubber coatings from substrates such as stainless steel. This limits the use of butyl rubber in biomedical applications because once delamination occurs it can cause major implications *in vivo* and is of particular relevance to coatings on drug-eluting stents. The adhesion properties of carboxylic acid functionalized butyl rubber (polymers 2.4a/b) are examined in this example compared to the adhesion properties of unfunctionalized butyl rubber (polymers 2.1a/b), epoxidized butyl rubber (polymers 2.2a/b and allylic alcohol functionalized butyl rubber (polymers 2.3a/b). The synthesis of polymers 2.2a/b, 2.3a/b, and 2.4a/b was performed on a 5-10 g scale as described above. Polymers 2.1a/b were obtained from LANXESS Inc. Surface adhesion or stickiness was determined by the amount of force required to remove a polymer sample from a substrate. A stainless steel substrate was selected due to the applicability of stainless steel to stents.

A Monsanto Tel-Tak™ Model TT-1 was used to determine the adhesion of uncured rubber samples to a stainless steel substrate surface. The adhesion test procedure was based upon ASTM D-429 Method A. This test determines the force required to achieve planar separation of an elastomer from a solid substrate. The compound being tested was initially sheeted from a two-roll mill and cut into 5" x 3" sample sheets of varying thickness (0.5 mm to 3.3 mm). The sample sheets were then pressed into a 12.7 cm x 7.6 cm mold containing square woven fabric using a 15 pound weight for 5 minutes at 100°C. The mold was backed by Mylar™ on one side and Teflon™ on the other in order to preserve the integrity of the sample surfaces. Stainless steel surfaces were cleaned and then preserved in glass jars containing ethanol, while the Teflon™ and Mylar™ were wiped down with ethanol directly prior to testing. All surfaces were cut into test strips measuring 6.35 mm x 50.8 mm. Tests were performed within 16 hours of specimen preparation. Care was taken to prepare and preserve the integrity of all specimen surfaces.

When performing the adhesion tests, the butyl rubber specimen was placed face up into the bottom of the sample holder of the Tel-Tak™ apparatus and the protective Mylar™ layer was removed. The stainless steel substrate surface was polished with ethanol and placed into the top sample holder above the specimen. Both sample holders were then placed into the apparatus. The surfaces were moved into contact with one another and a built-in timer set to 60 s was automatically activated. A contact pressure of 32 psi with a 450 g weight was applied using the apparatus. Following the 60 s contact time, the specimen and substrate surfaces were separated from one another at a speed of 2.54 cm per minute, while constantly maintaining a parallel relationship between the surfaces. The force required to separate the specimen from the surface was measured using a calibrated force gauge with a capacity of 2270 grams and a built-in indicator for the maximum value. The maximum force value could be read directly from the force gauge in pounds per square inch (psi). Tests were carried out in triplicate and the mean values were reported. The results are given in Table 2.

**Table 2**

| Butyl Rubber | Force to Separate (psi) | |
|---|---|---|
| | 2.2% Isoprene | 7% Isoprene |
| 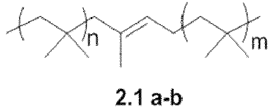 | 14.6 ± 0.3 | 12.1 ± 0.2 |
| 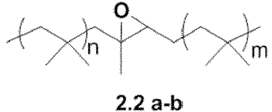 | 22.2 ± 2.2 | 25.2 ±1.6 |
| 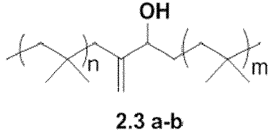 | 29.6 ± 0.6 | 20.5 ± 2.1 |
| 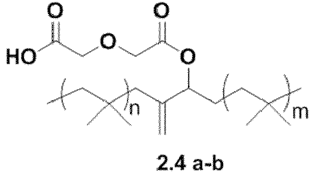 | 32.8 ± 1.3 | 28.3 ± 2.7 |

The adhesions of the commercially available butyl rubber 2.1a/b are well known and the various oxygenated butyl rubbers (2.2a/b, 2.3a/b, 2.4a/b) were tested and compared to 2.1a/b. All of the oxygenated butyl rubbers showed significant improvement in adhesion to the stainless steel substrate. The largest improvement in adhesion was seen with the carboxylic acid functionalized butyl rubber 2.4a, which took 32.8 ± 1.3 psi to separate. The adhesion of the high isoprene carboxylic acid functionalized butyl rubber 2.4b was also high, but reduction in flow of this polymer made it difficult to process the sample for this test, therefore the value observed for 3.4b may be lower than the actual adhesion value. The adhesion of the high isoprene carboxylic acid functionalized arborescent butyl rubber 2.4c was not tested but is expected to have similar adhesion improvements arising from the carboxylic acid moieties. The results demonstrate enhanced adhesion to stainless steel for carboxylic acid functionalized butyl rubber, making them promising materials for reducing delamination problems in stainless steel stents.

### Conjugation and Release of Paclitaxel to/from Carboxylic Acid Functionalized Butyl Rubber.

In this example, the anti-proliferative drug paclitaxel is covalently conjugated to a carboxylic acid functionalized butyl rubber to provide a sustained release in comparison to physically encapsulated drug.

### Synthesis of conjugates 3.0a/b

Scheme 3 illustrates the synthesis of the conjugates. Generally, synthesizing the paclitaxel-butyl rubber conjugates (3.0a/b) involves contacting paclitaxel with carboxylic acid functionalized butyl rubber (2.4a/b) in the presence of a dehydrating agent (e.g. a carbodiimide) and a catalyst (e.g. 4-dimethylaminopyridine (DMAP)) in a solvent. Various carbodiimide dehydrating agents may be used (e.g. dicyclohexylcarbodiimide (DCC), 1-ethyl-3-(3-dimethylaminopropyl)carbodiimide (EDC•HCl)), however EDC•HCl is preferred since the use of DCC produces byproducts that are more difficult to remove. Various catalysts may be used but nucleophilic catalysts are preferred.

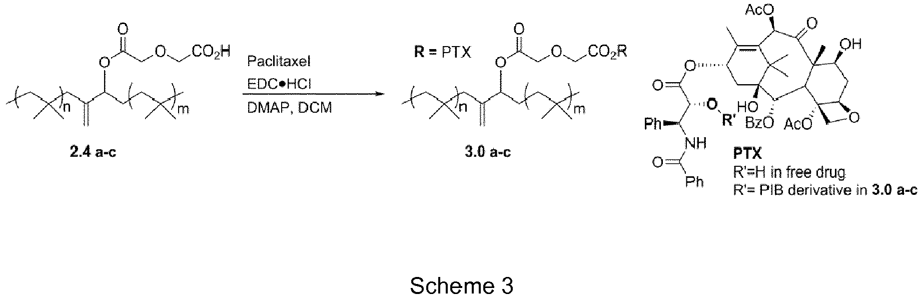

Conjugate 3.0a, with 2.2 mol% isoprene in the butyl rubber, was prepared as follows. A dried sample of 2.4a (10 g, 0.39 mmol CO₂H per gram of polymer) was dissolved in dry toluene and put under inert conditions. A solution of 1-ethyl-3-(3-dimethylaminopropyl) carbodiimide) (EDC) (1.25 eq. per CO₂H, 4.9 mmol, 0.76 g), and 4-dimethylaminopyridine (DMAP) (0.5 eq. per CO₂H, 1.95 mmol, 0.25 g), and N,N-diisopropylethylamine (2.0 eq. per CO₂H 1.2 mL, 6.8 mmol) were dissolved in dry CH₂Cl₂ and added to the 4.2a. The solution was stirred for 10-20 minutes prior to the addition of paclitaxel (PTX) (1.1 eq. per CO₂H, 4.29 mmol, 3.65 g) dissolved in CH₂Cl₂. The solution was left at room temperature overnight. The reaction mixture was then stripped of CH₂Cl₂ via rotary evaporation and then washed with deionized (DI) water, 1 M HCl and 1 M NaHCO₃, two times. Lastly the resultant solution was precipitated in ethanol and dried under vacuum. ¹H NMR (400 MHz, CDCl₃) δₚₚₘ 8.15 (d, *J* = 7.4 Hz, 2H, PTX), 7.76 (t, *J =* 7.1 Hz, 2H, PTX), 7.60 (t, *J* = 7.1 Hz, 1 H), 7.56-7.45 (m, 3H, PTX), 7.44-7.36 (m, 5 H, PTX), 7.34 (m, 1H, PTX), 7.15-7.10 (m, 1H, PTX), 6.30 (s, 1H, PTX), 6.29-6.20 (m, 1H, PTX), 6.08-6.01 (m, 1 H, PTX), 5.69 (t, *J* = 6.1 Hz, 1 H, PTX), 5.62-5.57 (m, 1 H, PTX), 5.29-5.19 (m, 1.1H, PIB), 5.13-5.06 (m, 1 H, PIB), 4.97 (d, *J* = 9.1 Hz, 1 H, PTX), 4.91 (m, 2H, PIB), 4.44 (m, 1 H, PTX), 4.37-4.12 (m, 6H, IB/PTX), 3.82 (*pseudo-*d*, J* = 6.5 Hz, 1 H, PTX), 3.68 (t, *J* = 6.4 Hz, 1 H, PTX), 2.62-2.52 (m, 1 H, PTX), 2.49-2.46 (s, 3H, PTX), 2.41-2.35 (m, 1 H, PTX), 2.26 (m, 2H, PTX), 2.22 (s, 3H, PTX) 1.96-1.92 (m, 4H, PTX), 1.69 (s, 3H, PTX) 1.42 (s, 315H, PIB), 1.26-0.91 (PIB, m, 950H, PIB/PTX). IR (thin film on NaCl, chloroform) 1232, 1367, 1390, 1475, 1670, 1737, 2960 cm⁻¹. SEC: M_{w} = 337,000 g/mol, PDI = 1.47.T_{g}=-62°C.

Conjugate 3.0b, with 7 mol% isoprene in the butyl rubber, was prepared as described above for 3.0a except that this polymer contained 1.2 mmol CO₂H per gram of polymer and thus the amounts of EDC, DMAP, DIPEA and PTx were increased to retain these reagent ratios at 1.25, 0.5, 2.0 and 1.1 equivalents per CO₂H, respectively. ¹H NMR (400 MHz, CDCl₃) 8.15 (d, *J* = 7.3 Hz, 2H, PTX), 7.74 (d, *J* = 6.4 Hz, 2H, PTX), 7.60 (t, *J* = 7.2 Hz, 1 H, PTX), 7.52 (t, *J* = 7.6 Hz, 2H, PTX), 7.49 (t, *J* = 7.6 Hz, 1 H, PTX), 7.44-7.31 (m, 8H, PTX), 7.02-6.96 (m, 1H, PTX), 6.29 (s, 1H, PTX), 6.28-6.20 (m, 2H, PTX), 6.08-6.00 (m, 1 H, PTX), 5.69 (d, *J =* 6.8 Hz, 1 H, PTX), 5.62-5.56 (m, 1 H, PTX), 5.28-5.19 (m, 1.4H, PIB), 5.12-5.07 (m, 1.4H, PIB), 4.97 (d, *J* = 9.4 Hz, 1.2H, PTX), 4.91-4.89 (m, 1.6H, PIB), 4.47-4.41 (m, 1 H, PTX) 4.40-4.10 (m, 9H, PTX/PIB), 3.82 (d, *J* = 6.5 Hz, 1 H, PTX), 3.70-3.64 (m, 1 H, PTX), 2.62-2.52 (m, 1 H, PTX), 2.52-2.44 (m, 4H, PTX), 2.42-2.34 (m, 2H, PTX), 2.23 (s, 3H, PTX), 1.94 (bs, 4H), 1.68 (bs, 3H, PTX), 1.41 (s, 112H, PIB), 1.29-0.87 (m, 400H, PTX/PIB); IR: 1232, 1367, 1390, 1475, 1670, 1737, 2960 cm⁻¹; SEC: M_{w} =501,400 g/mol, PDI = 2.66. T_{g} = -56 °C.

Conjugate 3.0c with 6 mol% isoprene in the arborescent butyl rubber, was prepared as described for 3.0a except that this polymer contained 1.1 mmol CO₂H per gram of polymer and thus the amounts of EDC, DMAP, DIPEA and PTx were increased to retain these ratios at 1.25, 0.5, 2.0 and 1.5 equivalents per CO₂H respectively. ¹H NMR^{*} (600 MHz, CDCl₃) δ 8.14 (bd, J = 6.4 Hz, 0.055H), 7.75 (bd, J = 6.9 Hz, 0.051 H), 7.63-7.58 (m, 0.027H), 7.53-7.47 (m, 0.17H), 7.45-7.33 (m, 0.19H), 7.22-7.19 (m, 0.062H), 7.10-7.05 (m, 0.027H), 6.31-6.20 (m, 0.057H), 6.07-5.97 (m, 0.026H) 5.70-5.65 (m, 0.028H), 5.64-5.56 (m, 0.032H), 5.29-5.17 (m, 0.067H), 5.15-5.08 (m, 0.061 H), 4.99-4.87 (m, 0.16H), 4.49-4.04 (m, 0.53H), 3.83-3.78 (m, 0.15H), 2.69-2.51 (m, 0.034H), 2.50-2.42 (m, 0.099H), 2.41-2.38 (m, 0.036H), 2.25-2.23 (m, 0.22H), 1.97-1.80 (m, 0.18H), 1.69 (s, 0.29H), 1.47-1.33 (m, 29.7H), 1.11 (s, 92H); SEC: M_{w} = 234 kDa, PDI = 1.82. IR (thin film on NaCl, chloroform): 923, 949, 1231, 1366, 1389, 1472, 1645, 1743, 2952 cm⁻¹; T_{g} = -65°C.

Conjugation of paclitaxel to the carboxylic acid functionalized butyl rubber was successful as confirmed by the ¹H NMR data, which shows characteristic paclitaxel and butyl rubber peaks and the disappearance of a peak at 4.81 ppm, which corresponds to the proton adjacent to the paclitaxel hydroxyl group at which conjugation occurs. After conjugation this peak shifts to 5.60 ppm. Furthermore the paclitaxel peak at 5.80 ppm, which corresponds to the proton adjacent to the benzyl ring, shifts to 6.05 ppm. Based on ¹H NMR spectroscopy, the conversion was approximately 95% for 3.0a, 85% for 3.0b, and 50% for 3.0c. Conjugate 3.0a contains about 24 wt% paclitaxel, 3.0b contains about 48 wt% paclitaxel, and 3.0c contains about 38 % paclitaxel which is consistent with the relative isoprene contents and reaction conversions.

### Release profiles of 3.0a-c

To study paclitaxel release profiles, thin films of the conjugates 3.0a-c and of unconjugated carboxylic acid functionalized butyl rubbers 2.4a-c were prepared. To prepare the films, 100 mg/mL solutions (in CH₂Cl₂) of the polymers were prepared. For samples involving physical encapsulation (2P, 7P, ArbP) rather than conjugation, the paclitaxel (PTX) was added next to obtain comparable weight percentages (i.e. 24 wt%, 48 wt%, 38 wt%). Also, two examples of styrene-isobutylene-styrene (SIBS) triblock copolymer with different amounts of styrene content (S1-10% and S2-20%) and 24 wt% of physically encapsulated paclitaxel were used as a comparison with the other systems.

A 100-µL aliquot of each of the polymer solution was drop cast onto a polished stainless steel plate (3 cm x 1 cm), the surfaces of the stainless steel plates having been polished with a bench-top grinder to give a smooth surface. The samples were dried under reduced pressure prior to the release study. Each sample was prepared and studied in triplicate. Table 3 provides a description of the samples.

**Table 3**

| Sample Name | Polymer Composition | PTx wt.% | PTx immobilization |
|---|---|---|---|
| 2C | 3.0a | ~24 | Covalent |
| 7C | 3.0b | ~48 | Covalent |
| ArbC | 3.0c | ~38 | Covalent |
| 2P | 2.4a | 24 | Physical |
| 7P | 2.4b | 48 | Physical |
| ArbP | 2.4c | 38 | Physical |
| S1 | SIBS - 10% styrene | 24 | Physical |
| S2 | SIBS - 20% styrene | 24 | Physical |

The release study was performed in 0.01 M phosphate buffered solution of pH = 7.4. The final buffer also contained 0.138 M NaCl, 0.0027 M KCl and 0.05% Tween™ 20 as a surfactant to help solubilize paclitaxel. The stainless steel plates were placed in a vial containing 10 mL of buffered solution. The solution was maintained at 37°C and the buffer was removed every 7 days for analysis of paclitaxel and replaced with fresh medium. Due to the low concentrations of paclitaxel released, the release medium was concentrated from 10 mL to 2 mL prior to HPLC analysis. The water was removed via lyophilization and the solid was re-dissolved in 2 mL of 80:20 water:acetonitrile.

HPLC analysis for paclitaxel was done with a Waters Separations Module 2695, a Photodiode Array Detector (Waters 2998) and a Nova-Pak C18 4 µm (3.9 mm x 150 mm) column connected to a C18 guard column. The PDA detector was used to monitor paclitaxel at 228 nm. Paclitaxel separation was obtained using a gradient method with Solvent A (5% acetonitrile in water) and Solvent B (80% acetonitrile, 0.1% H₃PO₄ in water) flowing at 1 mL/min. Gradient of Solvent A 65% was decreased to 30% over 10 min, and increased back to 65% over the next 5 min where the column was allowed to equilibrate over another 5 min. The calibration curve was obtained from paclitaxel (LC Laboratories, >99%, P-9600) standard solutions. Stock solutions of 1000 µg/mL, 100 µg/mL and 50 µg/mL of paclitaxel in acetonitrile were prepared. The stock solutions were used to make standard solutions of 25, 20, 15, 10, 7.5, 5, 2, 1, 0.5 µg/mL in 20:80 acetonitrile:PBS solution. Standards were filtered and injected at 100 µL using the above instrument method. Samples were prepared in a 20:80 acetonitrile:PBS solution, filtered through 0.2 µm filters and injected at 100 µL using the same conditions as described above. The limit of detection of paclitaxel was determined to be 0.02 µg. Table 4 provides the mass of PTX released (in µg) from polymer films at different time points. The standard deviation on three measurements is provided.

**Table 4**

| | 7 days | 14 days | 21 days | 28 days | 35 days |
|---|---|---|---|---|---|
| 2C | 0.40±0.03 | 0.80±0.02 | 1.21±0.03 | 1.63±0.04 | 2.06±0.04 |
| 7C | 0.30±0.02 | 0.70±0.02 | 1.30±0.03 | 1.65±0.03 | 2.10±0.03 |
| ArbC | 0.0±0.0 | 1.4±0.4 | 2.7±0.9 | 3.3±0.4 | 4.4±0.9 |
| 2P | 2.3±0.7 | 6.0±0.4 | 9.0±0.4 | 12.3±0.4 | 14.4±0.8 |
| 7P | 4±2 | 8±1 | 12±2 | 15.2±0.4 | 18±1 |
| ArbP | 0.5±0.2 | 5±2 | 6±2 | 6±2 | 8±2 |
| S1 | 5±4 | 12±7 | 20±2 | 27±4 | 39±4 |
| S2 | 4±3 | 8±3 | 10±5 | 13±6 | 16±6 |

Fig. 1 shows the paclitaxel release profile for the samples tested. The profile is shown in cumulative mass of paclitaxel released and it should be noted that some degradation products of paclitaxel were also seen in the HPLC trace, for example the known epimer. The covalently bound paclitaxel samples showed a sustained, slow release in comparison to the physically immobilized samples. The covalently bound paclitaxel samples also showed slower release in comparison to the SIBS samples. As observed in the prior art, the introduction of hydrophilic blocks increases the burst release of paclitaxel. However, this issue does not appear for the present covalently bound conjugates. The slow sustained release exhibited by the paclitaxel conjugated carboxylic acid functionalized butyl rubber samples eliminates burst release of drug. Despite the slow release of paclitaxel from films of the covalent conjugates, the drug was still able to prevent the adhesion and proliferation of cells on the film, which is important for their application as polymer-drug conjugates in drug eluting stents (Fig. 2 and Fig. 3).

### Procedure for cell growth on surfaces

C2C12 cells were maintained at 37 °C and 5% CO₂ in Dulbecco's Modified Eagle Medium (Invitrogen) supplemented with 10% fetal bovine serum (Invitrogen) and supplemented with 1% Glutamax (100x) solution and 1% Penstrep (100x). First, microscope glass cover slips (circular, 25 mm diameter) were coated with a minimum layer of polymer by applying a 35 mg/mL solution of polymer in toluene and allowing the solvent to dry completely. The surfaces were sterilized by submersion in 70% ethanol, and were then left to dry completely under reduced pressure for 96 hours. The sterilized samples were placed in the wells of a 6-well plate and 5 × 10⁵ cells in 2 mL of cell culture medium were seeded onto each surface. The samples were incubated for 48 hours, then fixed with 4% paraformaldehyde solution for 10 min. The samples were washed twice with phosphate-buffered saline (PBS) (Invitrogen) at pH 7.2, and then treated with 2 mL of acetone at -20 °C for 5 minutes to permeabilize the membrane. After that, they were washed again with PBS, stained with Alexa Fluor 568 phalloidin (Invitrogen) and DAPI (Invitrogen) following the manufacturer's directions. The samples were washed again with PBS and placed face down onto glass microscope slides with ProLong® Gold Antifade Reagent (Invitrogen) and sealed. Confocal images were obtained using a confocal laser scanning microscope (LSM 510 Duo Vario, Carl Zeiss) using a 20x objective and excitation wavelengths of 405 (DAPI) and 578 nm (phalloidin). Cell were counted using Image Pro Plus software on 5 different images. Statistical analyses (ANOVA followed by Tukey's test) were performed using the software Excel.

### Adhesion of 3.0a/b to stainless steel

The films of the samples 2C, 7C, 2P, 7P, S1 and S2 from above were examined visually, by scanning electron microscopy (SEM) and by atomic force microscopy (AFM) for delamination of the polymer coating from the stainless steel substrate during the 35 days of the release study. The covalently bound samples (2C, 7C) exhibited enhanced film adhesion to the stainless steel substrate in comparison to the controls (2P, 7P, S1, S2). As shown in Fig. 4, delamination of the physically immobilized samples was visually observed over the 35 day incubation period, whereas no delamination was visually observed for the covalently bound sample (2C, 7C, ArbC). SEM (Fig. 5) showed that the films were initially smooth and uniform, but after 35 days in the release medium, the integrity of the SIBS-based films (S1, S2) and the physically immobilized samples (2P, 7P) had decreased drastically. The surfaces of the SIBS-based films (S1, S2) became non-homogenous with visible surface degradation. The films of the physically immobilized samples (2P, 7P, ArbP) developed cracks all over the polymer surface. However, films of the covalently bound samples (2C, 7C) appeared to remain intact after 35 days. This was also observed by atomic force microscopy (AFM), which showed significant erosion of the films with noncovalent PTX in comparison with the conjugates (Fig. 6).

### Toxicity

Toxicity studies were also performed using the MTT assay to evaluate whether films of 3.0a-c on stainless steel would release toxic levels of paclitaxel or any other leachate during a defined incubation period in cell culture media.

Preparation of leachate: Test samples were melt-pressed to a thickness of 0.4 mm. The melt pressed film was then cut into squares of 1 cm x 1 cm. Samples were sterilized by washing with 70% ethanol and subsequently dried for 2 h under UV light. Samples were placed in Petri dishes and incubated in 2 mL of Dulbecco's Modified Eagle Medium (DMEM, Invitrogen) supplemented with 10% fetal bovine serum (Invitrogen) and supplemented with 1% Glutamax (100x) solution and 1% Penstrep (100x) in an incubator at 37°C for 24 h. The leachate was then removed and passed through a 0.2 µm filter.

MTT assay: C2C12 mouse myoblast cells were seeded in a Nunclon™ 96-well U bottom transparent polystrol plate to obtain 10,000 cells/well in 100 µL of DMEM containing serum, glutamax and antibiotics as described above. The cells were allowed to adhere in a 5% CO₂ incubator at 37°C for 24 hr. The growth medium was then aspirated and was replaced with either the positive control (sodium dodecyl sulfate (SDS) in the cell culture medium at concentrations of 0.2, 0.15, 0.10, or 0.05 mg/mL), the negative control high density polyethylene (HDPE)), serial two-fold dilutions of the leachate, or just the medium. The cells were then incubated at 37°C (5% CO₂) for 24 h. The medium was then aspirated and replaced with 110 µL of fresh medium containing 0.5 mg/mL (3-(4,5-dimethylthiazol-2-yl)-2,5-diphenyltetrazolium bromide) (MTT) reagent. After 4 h of incubation (37°C, 5% CO₂), the MTT solution was carefully aspirated and the purple crystals were dissolved by addition of 50 µL of spectroscopic grade dimethylsulfoxide (DMSO). After shaking (1 second, 2 mm amp, 654 rpm), the absorbance of the wells at 540 nm was read using an M1000-Pro plate reader (Tecan). The absorbance of wells not containing cells but treated by all of the above steps was subtracted as a background and the cell viability was calculated relative to wells containing cells that were exposed to just culture medium. No (0%) cell viability was detected for the cells exposed to the highest concentrations of the positive control sodium lauryl sulfate, confirming the sensitivity of the assay.

As a known non-toxic control, high-density polyethylene (HDPE) was the negative control. Sodium dodecyl sulfate (SDS) was used as a positive control and high toxicity (< 10% cell viability) was detected at 0.2 µg/mL, demonstrating the efficacy of the assay in detecting toxicity. To detect the leaching of any potentially toxic molecules, the paclitaxel conjugated carboxylic acid functionalized butyl rubbers (2C, 7C, ArbC), the physically immobilized samples (2P, 7P), SIBS samples with physically immobilized paclitaxel (S1, S2), SIBS samples without paclitaxel (SIBS1, SIBS2) and carboxylic acid functionalized butyl rubbers 2.4a-c without paclitaxel were also tested.

As shown in Fig. 7, most of the materials did not lead to any detectable toxicity, with only a few of the films exhibiting modest toxicity at the highest leachate concentrations. The films that exhibited modest toxicity were the physically immobilized paclitaxel materials (2P, 7P) as well as the SIBS materials (S1, S2), which released paclitaxel the most rapidly in the release study. It should be noted that although paclitaxel is a very toxic drug, the amounts of paclitaxel released over the 24 hour incubation period would still be extremely small. Polymers without paclitaxel did not exhibit any toxicity in this assay. These results further support the use of carboxylic acid functionalized butyl rubbers and polymer-drug conjugates based thereon for biomedical applications.

The novel features will become apparent to those of skill in the art upon examination of the description. It should be understood, however, that the scope of the claims should not be limited by the embodiments, but should be given the broadest interpretation consistent with the wording of the claims and the specification as a whole.

## Claims

1. A polymer-drug conjugate comprising at least one active agent conjugated through a carboxylic acid moiety to a copolymer derived from at least one isoolefin monomer and at least one copolymerizable monomer, the copolymerizable monomer comprising at least one multiolefin monomer, a β-pinene monomer or a mixture thereof.

2. The conjugate according to claim 1 having Formula (I):
Polymer-Rc-Ag (I)
wherein
Polymer is the copolymer derived from at least one isoolefin monomer and at least one copolymerizable monomer,
Ag is the active agent, and
Rc comprises a ring-opened residue of a saturated cyclic anhydride.

3. The conjugate according to claim 2, wherein the saturated cyclic anhydride had a five- or six-membered ring before ring-opening.

4. The conjugate according to claim 2, wherein Rc is

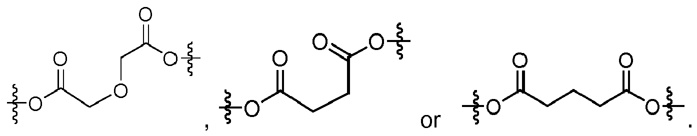

5. The conjugate according to claim 2, wherein Rc is

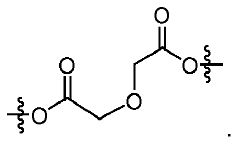

6. The conjugate according to any one of claims 1 to 5, wherein the at least one isoolefin monomer comprises polyisobutylene.

7. The conjugate according to any one of claims 1 to 6, wherein the copolymerizable monomer comprises at least one conjugated diene.

8. The conjugate according to any one of claims 1 to 6, wherein the copolymerizable monomer comprises isoprene.

9. The conjugate according to any one of claims 1 to 8, wherein the copolymer is linear.

10. The conjugate according to any one of claims 1 to 8, wherein the copolymer is arborescent.

11. The conjugate according to any one of claims 1 to 10, wherein the active agent comprises a microtubule stabilizing drug.

12. The conjugate according to any one of claims 1 to 10, wherein the active agent comprises paclitaxel.

13. Use of a therapeutically effective amount of a polymer-drug-conjugate as defined in any one of claims 1 to 12 for treating or preventing a disease or condition in a subject.

14. Use of a therapeutically effective amount of a polymer-drug-conjugate as defined in any one of claims 1 to 12 for manufacturing a medicament for treating or preventing a disease or condition in a subject.

15. A method of treating or preventing a disease in a subject comprising administering a therapeutically effective amount of a polymer-drug-conjugate as defined in any one of claims 1 to 12 to a subject in need of the treatment or prevention.

16. A stent comprising a mesh tube coated with a polymer-drug conjugate as defined in any one of claims 1 to 12.

17. The stent according to claim 16, which is a vascular stent

18. The stent according to claim 16 or 17, wherein the tube comprises stainless steel.

19. The stent according to claim 18, wherein the polymer-drug conjugate has an adhesion force to the stainless steel of about 15 psi or greater.
